# EUROPEAN PATENT APPLICATION

(11) **EP 4 545 020 A1**
(43) Date of publication of application: **30.04.2025**
(21) Application number: 24208624.7
(22) Date of filing: 24.10.2024
(51) Int. Cl.: A61B 17/16

(54) **SURGICAL BUR WITH IMPROVED CUTTING AND METHOD OF IMPROVING CUTTING EFFECTIVENESS**

(30) Priority: 24.10.2023 US 202363592823 P; 31.07.2024 US 202418789926
(71) Applicant: Medtronic PS Medical, Inc., Fort Worth, Texas 76137 (US)
(72) Inventor: HAUSER, Bret, Fort Worth, 76137 (US); BEAMON, Hubert, Fort Worth, 76137 (US); BARNES, Milton, Fort Worth, 76137 (US); MAHARJAN, Mahin, Fort Worth, 76137 (US); MALLA, Aayush, Fort Worth, 76137 (US); KULAS, John, Fort Worth, 76137 (US)
(74) Representative: Viering, Jentschura & Partner mbB Patent- und Rechtsanwälte

(57) **Abstract**

A surgical bur for use in cutting bone includes a stem adapted to selectively couple to an attachment tube of a micro-burring instrument assembly and is configured for selective rotation upon activation of the micro-burring instrument. The surgical bur includes a plurality of cutting flutes disposed at a distal end of the stem which defines a corresponding number of clearance surfaces disposed therebetween. Each of the cutting flutes includes a cutting edge and a trailing edge. One or more push-off elements is defined in the clearance surface between adjacent pairs of cutting flutes of the plurality of cutting flutes, the push-off elements being configured to contact bone during rotation of the surgical bur and being configured to deflect the surgical bur relative thereto and optimize the cutting efficiency and effectiveness of the surgical bur based on the rotational speed (RPM) thereof.

## Description

### FIELD

The present disclosure relates to surgical systems for bone cutting or bone shaping, and, more particularly, to surgical burs for cutting and shaping bone.

### BACKGROUND

A surgical instrument for cutting or shaping bone typically includes a motor housing with a grip, a surgical attachment, and a surgical tool. The surgical attachment and the surgical tool may be interchangeable with other surgical attachments and surgical tools. The surgical attachment connects to the motor housing and engages with a motor disposed therein configured to rotate, oscillate or otherwise move the surgical tool upon activation thereof. The surgical tool typically includes an elongated shaft and a surgical cutting head or bur attached at a distal end thereof (or integrally associated therewith). The elongated shaft extends from the surgical bur, engages with the surgical attachment and is axially rotated, oscillated or otherwise moved upon actuation of the motor.

Surgical burs are used to dissect, cut and/or shape bone during a surgical procedure. Various characteristics of surgical instruments or surgical burs are often designed for particular purposes to improve surgical performance. Such characteristics include cutting efficiency, stability, working length, and visibility. Improving one of these characteristics often negatively affects one or more of the other characteristics. For example, designing a surgical bur with an aggressive cutting flute may improve cutting efficiency, but at the same time, the same cutting flute may impede another aspect of the surgical procedure, e.g., cause unacceptable vibration.

Such surgical instruments may include an elongated shaft coupled to a motor to provide torque to the tool to rotate the surgical bur during an operation. An attachment assembly holds the tool relative to the motor typically by a collet having various gears and connection portions that transfer torque from the motor to the tool. The attachment assembly may provide various features, such as a bore diameter, length, angle, and the like to allow selecting the tool to be operated by the motor. Further, the attachment assembly may include stiffness modification and/or damping features such as damping members, thicknesses, and the like to minimize and/or reduce vibration at a tool tip (e.g., surgical bur) and caused by the tool felt and received by a user.

In some cases, uncontrolled vibration or what is commonly referred to as "tool chatter" can negatively affect the cutting performance of the surgical bur. Too little chatter may also cause the surgical bur to cut inefficiently. If tuned correctly, a controlled amount of tool chatter can optimize a surgical bur's cutting effect on tissue, e.g., bone or other hard tissue structures. This often requires manual adjustment of the surgical bur within the tool holder, adjustment of one or more dampening mechanisms or other control mechanism which can be tedious to the surgeon and/or require multiple modifications during a given procedure.

As a result, manufacturing a surgical bur with way to self-regulate tool chatter to more effectively and efficiently cut bone would be a significant improvement in the field.

### SUMMARY

Provided in accordance with aspects of the present disclosure is a surgical bur for use in cutting bone which includes a stem adapted to selectively couple to an attachment tube of a micro-burring instrument assembly and configured for selective rotation upon activation of the micro-burring instrument. A plurality of cutting flutes is disposed at a distal end of the stem, the plurality of cutting flutes defining a corresponding number of clearance surfaces disposed therebetween, each cutting flute of the plurality of cutting flutes including a cutting edge and a trailing edge. One or more push-off elements is defined in the clearance surface between one or more adjacent pairs of cutting flutes of the plurality of cutting flutes. The push-off element(s) is configured to contact bone during rotation of the surgical bur and configured to deflect the surgical bur relative thereto and optimize the cutting efficiency and effectiveness of the surgical bur based on the speed (RPM) of the surgical bur.

In aspects in accordance with the present disclosure, a corresponding plurality of push-off elements is disposed between the plurality of cutting flutes. In other aspects in accordance with the present disclosure, the plurality of push-off elements is configured to deflect the surgical bur based on at least an angle of rotation of the surgical bur or an angle of oscillation of the surgical bur to optimize the cutting efficiency and effectiveness of the surgical bur based on the speed (RPM) of the surgical bur.

In aspects in accordance with the present disclosure, the plurality of push-off elements is configured to deflect the surgical bur based on at least an angle of rotation of the surgical bur or an angle of oscillation of the surgical bur to optimize the cutting efficiency and effectiveness of the surgical bur based on the speed (RPM) of the surgical bur.

In aspects in accordance with the present disclosure, the plurality of push-off elements is non-cutting.

In aspects in accordance with the present disclosure, a series of push-off elements are disposed between one or more adjacent pair of cutting flutes of the plurality of cutting flutes.

In aspects in accordance with the present disclosure, the series of push-off elements disposed between one or more adjacent pair of cutting flutes of the plurality of cutting flutes are equally spaced between cutting flutes.

In aspects in accordance with the present disclosure, the series of push-off elements disposed between at least one adjacent pair of cutting flutes of the plurality of cutting flutes are unequally spaced between cutting flutes.

In aspects in accordance with the present disclosure, the plurality of push-off elements disposed between the adjacent pairs of cutting flutes of the plurality of cutting flutes are synchronized such that the surgical bur does not completely bottom out as the next approaching cutting flute in rotation comes into contact with bone resulting in a more angular and aggressive cut.

In aspects in accordance with the present disclosure, the plurality of push-off elements disposed between the adjacent pairs of cutting flutes of the plurality of cutting flutes are synchronized such that the surgical bur pushes off from the bone between each adjacent pair of flutes more laterally along the direction of rotation than vertically along a direction normal to the bone.

Provided in accordance with aspects of the present disclosure is a micro-burring instrument for cutting bone which includes a micro-burring assembly having: a motor housing including a motor disposed therein; an attachment tube operably coupled at a proximal end thereof to the motor housing and configured to selectively receive a surgical bur therein for use in cutting bone; and a collet for operably receiving a stem of the surgical bur such that the surgical bur is selectively rotatable upon activation of the motor. The surgical bur includes: a plurality of cutting flutes is disposed at a distal end of the stem, the plurality of cutting flutes defining a corresponding number of clearance surfaces disposed therebetween, each cutting flute of the plurality of cutting flutes including a cutting edge and a trailing edge. One or more push-off elements is defined in the clearance surface between one or more adjacent pairs of cutting flutes of the plurality of cutting flutes. The push-off element(s) is configured to contact bone during rotation of the surgical bur and configured to deflect the surgical bur relative thereto and optimize the cutting efficiency and effectiveness of the surgical bur based on the speed (RPM) of the surgical bur.

In aspects in accordance with the present disclosure, a corresponding plurality of push-off elements is disposed between the plurality of cutting flutes. In other aspects in accordance with the present disclosure, the plurality of push-off elements is configured to deflect the surgical bur based on at least an angle of rotation of the surgical bur or an angle of oscillation of the surgical bur to optimize the cutting efficiency and effectiveness of the surgical bur based on the speed (RPM) of the surgical bur.

In aspects in accordance with the present disclosure, the plurality of push-off elements is configured to deflect the surgical bur based on at least an angle of rotation of the surgical bur or an angle of oscillation of the surgical bur to optimize the cutting efficiency and effectiveness of the surgical bur based on the speed (RPM) of the surgical bur.

In aspects in accordance with the present disclosure, the plurality of push-off elements is non-cutting.

In aspects in accordance with the present disclosure, a series of push-off elements are disposed between one or more adjacent pair of cutting flutes of the plurality of cutting flutes.

In aspects in accordance with the present disclosure, the series of push-off elements disposed between one or more adjacent pair of cutting flutes of the plurality of cutting flutes are equally spaced between cutting flutes.

In aspects in accordance with the present disclosure, the series of push-off elements disposed between at least one adjacent pair of cutting flutes of the plurality of cutting flutes are unequally spaced between cutting flutes.

In aspects in accordance with the present disclosure, the plurality of push-off elements disposed between the adjacent pairs of cutting flutes of the plurality of cutting flutes are synchronized such that the surgical bur does not completely bottom out as the next approaching cutting flute in rotation comes into contact with bone resulting in a more angular and aggressive cut.

In aspects in accordance with the present disclosure, the plurality of push-off elements disposed between the adjacent pairs of cutting flutes of the plurality of cutting flutes are synchronized such that the surgical bur pushes off from the bone between each adjacent pair of flutes more laterally along the direction of rotation than vertically along a direction normal to the bone.

### BRIEF DESCRIPTION OF THE DRAWINGS

The above and other aspects and features of the present disclosure will become more apparent in view of the following detailed description when taken in conjunction with the accompanying drawings wherein like reference numerals identify similar or identical elements.
FIG. 1 is a perspective view of a micro-burring surgical instrument in accordance with aspects of the present disclosure;
FIG. 1A is a perspective view of an angled attachment assembly including a surgical bur disposed therein for cutting bone;
FIG. 2 is a plan view of an attachment assembly, in accordance with aspects of the present disclosure;
FIG. 3A is an internal, cross-sectional view of the attachment assembly of FIG. 2 taken along line 3A-3A;
FIG. 3B is an internal, cross-sectional view of an alternative attachment assembly of FIG. 2 taken along line 3A-3A;
FIG. 3C is an internal, cross-sectional view of an alternative attachment assembly of FIG. 2 taken along line 3A-3A;
FIG. 4 is an exploded view of the attachment assembly of FIG. 2;
FIG. 5 is an internal, cross-sectional view of an attachment tube along a longitudinal axis of the attachment tube in accordance with aspects of the present disclosure;
FIG. 6 is an internal, cross-sectional view of an attachment assembly in accordance with aspects of the present disclosure;
FIG. 7A is an enlarged, perspective view of a distal end of the surgical bur including a plurality of flutes disposed therearound for cutting bone;
FIG. 7B is a greatly-enlarged, cross-section of the surgical bur of FIG. 7A taken along line 7B-7B highlighting a push-off disposed between two adjacent flutes; and
FIG. 8 is a schematic illustration of an exemplary robotic surgical system configured for use with the surgical instrument of FIGS. 1 and 1A and the surgical bur of FIGS. 7A and 7B in accordance with aspects of the present disclosure.

Corresponding reference numerals indicate corresponding parts throughout the several views of the drawings.

### DETAILED DESCRIPTION

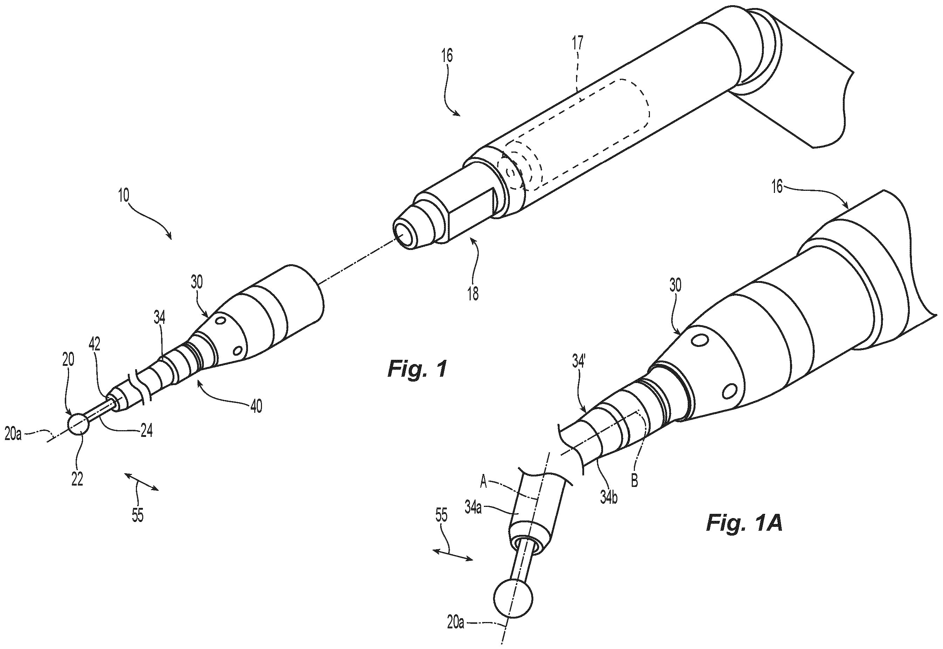

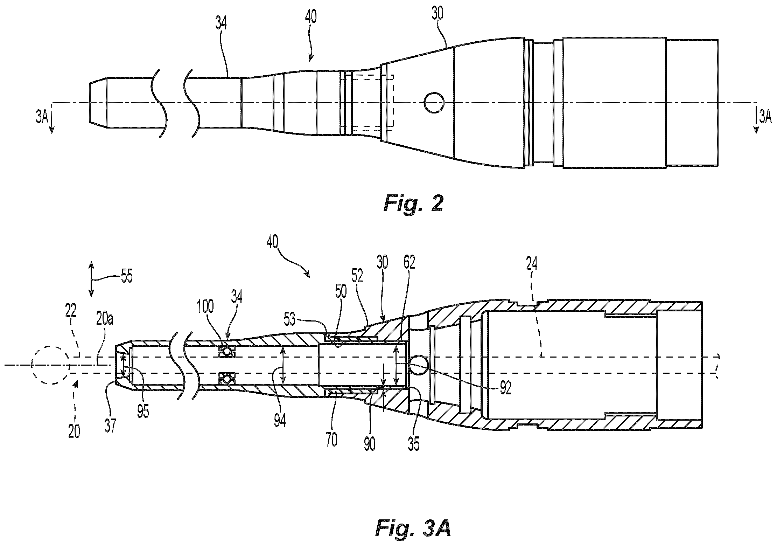

Example embodiments will now be described more fully with reference to the accompanying drawings and with initial reference to FIGS. 1, 1A, and 2, an instrument assembly 10 is illustrated. Instrument assembly 10 can be similar to one or more known instrument assemblies used to resect tissue (e.g., bone or other hard tissue structures), for example the Straightshot^{®} M4 Microdebrider powered handpiece or the Midas Rex^{®} Legend EHS Stylus" High-Speed Surgical Drill, which may be selectively used for ear-nose-throat (ENT) or neurosurgery, sold by Medtronic. Instrument assembly 10 includes a motor housing 16 that extends along an axis 20a and may have various angled or ergonomically shaped portions as described below.

Housing 16 includes a motor 17 disposed therein having a collet assembly 18 composed of various gears and components to operably connect the motor 17 with a surgical tool, e.g., surgical bur 20. Bur 20 includes a working end 22 and a stem 24. The working end 22 may include a surgical bur, a surgical drill bit, a resection tool, or other appropriate working surgical tool. Motor 17 is configured to regulate torque to the working end 22 through the shaft 24 via an interconnection within the collet 18.

Instrument assembly 10 also includes an attachment assembly 40 including an attachment base or housing 30 and an attachment tube 34 including a bore 42 defined therethrough that extends from a proximal end 35 to a distal end 37. The attachment base 30 and the attachment tube 34 are selectively removeable from the collet 18 during a selected procedure via any known removeable coupling in the art. Attachment tube 34 may include a proximal portion B and a distal portion A that are disposed at an angle relative to one another depending upon a particular surgical purpose. The angle between the portions A and B may be selected based upon a selected procedure and may further include movable interconnections to provide power to the tool 20 from the motor 17 through the angled region. Examples of how these interconnections may be formed are disclosed in commonly-owned U.S. Patent No. 10,527,478, the entire contents of which being incorporated by reference herein.

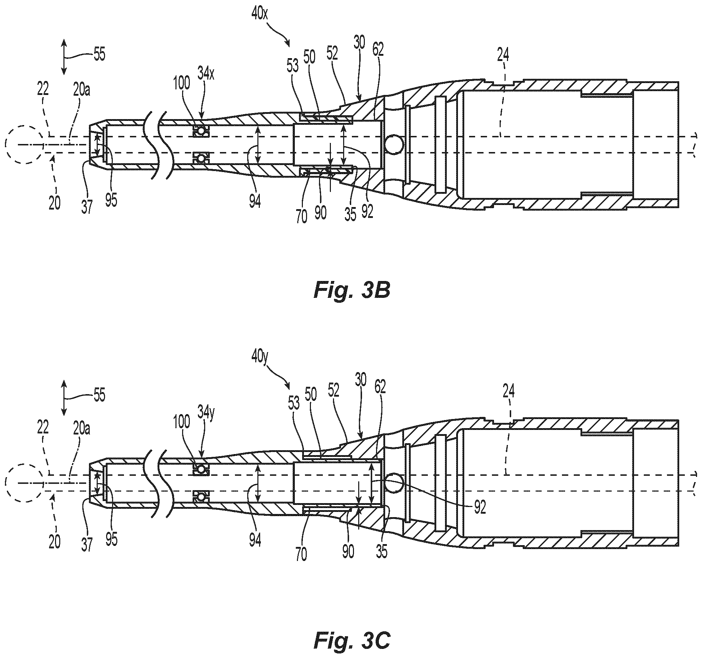

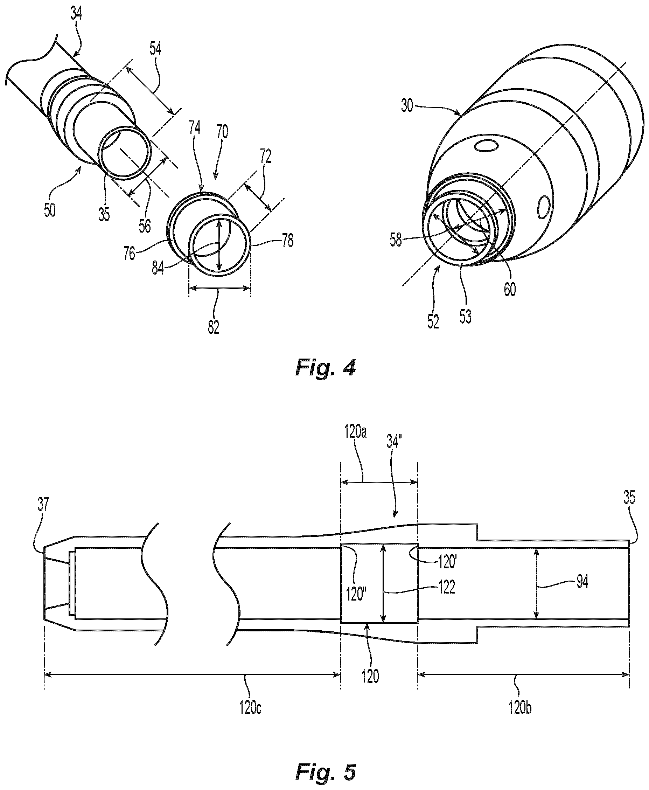

With continuing reference to FIG. 3A and additional reference to FIGS. 3B, 3C, and 4, the attachment assembly 40 is discussed in greater detail. Attachment assembly 40 includes a generally cylindrical exterior attachment tube 34 that is engaged within the attachment base 30. Inner bore 42 is formed by an internal annular wall that varies in diameter along the length of the bore 42 at selected regions, e.g., proximal end 35 has a greater diameter than distal end 37.

As mentioned above, the attachment tube 34 includes a connection region 50 that is received within receiving section 52 proximate the proximal end 35. Connection region 50 may have an external thread to engage an internal thread in the receiving section 52. Other coupling mechanisms are envisioned such as press-fit, brazing, and/or welding, a threaded connection, an adhesive, a stake, or other appropriate connections may be used to connect the attachment tube 34 to the attachment base 30. Further, the attachment tube 34, in various embodiments, may be coupled both directly and indirectly to the attachment base 30 (illustrated in FIG. 3A), may be only indirectly coupled to the attachment base 30 (illustrated in FIG. 3B), or may be coupled directly to the attachment base 30 (illustrated in FIG. 3C).

The receiving section 52 of the attachment base 30 can include a first inner diameter 58 that may be greater than the outer diameter 56 to receive an intermediate or damping member 70 (also illustrated in FIG. 4). The receiving section 52 may further include a second inner diameter 60 that may be substantially equivalent to or slightly larger than the outer diameter 56 of the tube connection section 50. The relationship of the inner diameter 58 and the outer diameter 56 of the damping member 70 may allow or form a press-fit connection between the damping member 70 and the attachment base 30. When assembled, therefore, the connection region 50 of the attachment tube 34 may be generally concentric with the receiving section 52 of the attachment base 30.

The tool 20 is configured to rotate around the axis 20a via torque being transmitted to the tool 20 from the motor 17 within the motor assembly 16. What is commonly referred to as "tool chatter" may be caused by movement of the tool 20 and/or the instrument assembly 10 in a direction that is normal to the axis 20a (see double-head arrow 55). Rotation of the tool 20 may cause vibration in any lateral direction relative to the axis 20a. Operating the tool 20 at a selected rotational speed may also reduce vibrations, such as lateral movements away from the axis 20a by the tool 20 and/or the attachments assembly 40.

As illustrated in FIG. 3A, the connection region 50 of the attachment tube 34 may extend to a direct bonding region 62 to allow for direct connection of the attachment tube 34 to the attachment base 30. For example, in the direct bonding region 62, an external thread on the tube connection section 50 can engage inner threads that are formed on the inner diameter 60 of the connection region 52 at the direct bonding region 62. The attachment tube 34 may then be threaded to the attachment base 30 to allow for direct connection of the attachment tube 34 to the attachment base 30. It is understood, however, that other connections and bonding of the attachment tube 34 to the base 30 can be made. For example, selected adhesive materials, press fit, and other connections can be formed between the attachment tube 34 and the attachment base 30.

With reference to FIG. 3B, it is understood that the attachment tube 34 need not be directly connected to the attachment base 30. Rather, an attachment assembly 40x may have an attachment tube 34x. The attachment tube 34x may be substantially similar to the attachment tube 34, discussed above. The attachment tube 34 may have the connection portion 50 that may only contact the damping member 70 within the attachment base 30. Thus, the attachment tube 34 may be interconnected with the attachment base 30 via the damping member 70 and not have a direct contact to the attachment base 30, at least within the connection region 52. The attachment tube 34 may, however, contact an external surface 53 of the attachment base 30.

As illustrated in FIG. 3C, the attachment tube 34 need only directly contact and connect to the attachment base 30. Connecting the attachment tube 34 only directly to the attachment base 34 may be a selected alternative to including the damping member 70. An attachment assembly 40y may have an attachment tube 34y. The attachment tube 34y may be substantially similar to the attachment tube 34, discussed above. The attachment tube 34y may have the connection portion 50 that may only contact the attachment base 30, including at the exterior surface 53 and the direct bonding region 62. Thus, a gap 71 may be formed if the internal diameter 58 is maintained. It is understood, however, that the connection region 52 may include only the internal diameter 60 such that no gap 71 is present (FIG. 4).

With continuing reference to FIGS. 1-3C and additional reference to FIG. 4, the damping member 70 may extend a length 72 between an outer or distal end 7 4 that may include a lip or edge 76 and a proximal end 78. The damping member 70 may be generally cylindrical and have a substantially annular wall that forms an external diameter 82 and an internal diameter 84. The external diameter 82 may be substantially equivalent to or slightly smaller than the inner diameter 58 of the base connection region 52. Therefore, the damping member 70 may be press fit and held in the connection region 52. It is further understood, however, that the damping member 70 may be bonded to the attachment base 30 with an adhesive, a threaded connection, or other appropriate connection. The inner diameter 84 may be formed to receive the connection region 50 and may be substantially equivalent to or equal to the inner diameter 60 of the connection region 52.

It is further understood that the damping member 70, according to various embodiments, may be molded or formed onto the attachment base 30, the attachment tube 34, or both. For example, the damping member 70 may be injection molded into the connection 50 of the attachment tube 34 or the connection 52 of the attachment base 30. Thus, the connection of the damping member 70 may be made relative to the attachment assembly 40 in selected manners.

The damping member 70 may further have a thickness (formed by the difference between the internal diameter 84 and the outer diameter 82), length 72, material, or other features selected based upon operation parameters of the attachment assembly 40. The operational parameters may include a rotational speed of the tool 20, the length of the attachment tube 34, the angle of the attachment tube 34', the diameter of the shaft 24, etc. Still further, the damping member 70 may be selected from a material with a relatively high loss factor (i.e., a material with the ability to absorb and/or transform kinetic energy to another form of energy), but also suitable for a selected procedure. For example, a viscoelastic polymer may provide a selected loss factor while being able to withstand repeated heat and steam sterilization and/or chemical sterilization for an operative procedure on a human patient. The damping member 70 may, for example, be formed of an elastomer, a silicone rubber, FKM (as determined by ASTM D1418) or similar fluoroelastomer, chlorobutyl elastomer, or other polymer or elastomer material. One example includes Viton^{®} fluoropolymer elastomer sold by E.I. du Pont de Nemours and Company or The Chemours Company having a place of business at Wilmington, Delaware.

As discussed above, in various embodiments, the tube connection region 50 can be fitted within the inner diameter 84 of the damping member 70 and placed within the connection region 52 of the attachment base 30. According to various embodiments, as discussed above, the tube connection region 50 may extend no longer than the length 72 of the damping member 70. It is understood, however, that the attachment tube 34 may connect directly and only to the attachment base 30.

The damping member 70 may include the various characteristics discussed above to tune the vibration of the instrument assembly 70, such as at a portion held by a user at the base 30 or motor housing 16, and chatter at the tool tip 22. The damping member 70 may be tuned to dampen vibration and/or tool chatter by a selected amount by selecting the thickness, length, material, location, etc. The reduced vibration and tool chatter may ensure a precise resection or operation of the assembly 10.

Tuning the vibration may occur without or in addition to the damping member 70 and characteristics of the damping member 70. For example, the attachment region 50 of the attachment tube 34 may include a selected wall thickness 90 to assist in reduction of vibration during operation of the tool 20. As illustrated in FIGS. 3A, 3B, and 3C, the tube connection region 50 can include the wall thickness 90 that can be selected according to various features and limitations of the system. The thickness 90 of the connection region 50 may be formed by increasing an internal diameter of the connection tube 34 on the selected portion of its length.

For example, as illustrated in FIGS. 3A, 3B, and 3C, the thickness 90 is formed by an internal diameter 92 in a first portion of the attachment tube 34 while an internal diameter 94 is formed in a second portion of the attachment tube 34. The attachment tube 34 may include other internal diameters, such as a terminal internal diameter 95 where the tool 20 extends from the attachment tube 34 near the working end 22 of the tool 20. It is understood, the attachment tube 34 may be coupled to the attachment base 30 without the damping member 70. The selected two internal diameters 92 and 94 forming the thickness may, therefore, alone provide a vibration reduction feature.

Reducing the vibration, therefore, may be created using one or more of the selected thickness, axial length of a region with a selected thickness, damping member, etc. Creating a selected vibration design limitation, including reducing vibrations with a selected feature, may be selected by reducing or forming a selected thickness at a selected location. The reduced or formed thickness may be by cutting or forming an internal diameter or cutting into an outer surface of the attachment tube 34.

As briefly discussed above, during operation, the tool 20 may rotate around the axis 20a in selected directions and may oscillate. During rotation and oscillation of the tool 20, vibrations may be induced in the attachment assembly 40. The vibrations may be due to rotation of the tool 20 or operation of a motor 17 in the motor housing or motor assembly 16. The vibrations may be reduced by either or both (i.e., combined) the thickness 90 of the attachment tube 34 and the damping member 70. As noted above, the reduction in vibration and tool chatter may be achieved by forming the selected thickness 90 at any appropriate axial positional along the attachment tube 34. The tool shaft 24 may ride in one or more bearings 100 that are connected within the internal diameter 94 of the attachment tube 34. Therefore, rotation of the tool 20 may be radially guided by the attachment tube 34. This may transmit vibrations to the attachment housing 30, the attachment tube 34, and may be transmitted to motor 16 or other portion grasped by a user.

The attachment tube 34, therefore, includes the selected thickness 90 and may be tuned relative to an operation of the tool 20 and geometries and configurations of the attachment tube 34 and/or the attachment base 30. For example, the internal diameter 94 of the bore 33 extending through the attachment tube 34, the length of the attachment tube 34, a geometry of the attachment base 30, a geometry of the attachment tube 34, such as the angle attachment tube 34', may all be considered when determining the selected thickness 90 of the attachment tube 34. Further, a size, including a thickness, such as the difference between the internal diameter 84 and the external diameter 82 of the damping member 70, may be selected for tuning vibrational reduction of the attachment assembly 40 and/or the tool assembly 10. Tuning of vibration felt by a user, such as a surgeon, and tuning of tool chatter (i.e., lateral movement of the tool head 22) may be based on various features, as discussed herein. Further, specifics of the features, including size, placement, etc. may be based on specifics of the selected attachment assembly. As discussed above, various attachment assemblies may be provided in various configurations such that the specifics of the tuning features may vary amongst the attachment assemblies.

Tuning, selecting an amount of or eliminating vibration and tool chatter may vary based on several considerations. For example and as illustrated in FIG. 5, another embodiment of an attachment tube 34" may include a selected thickness as a vibration reduction or tuning feature away from the terminal ends, including proximal terminal end 35. For example, the attachment tube 34" may include the internal diameter substantially along an entire length of the attachment tube 34". However, a vibration reducing or tuning feature 120 may be formed as a notch or a groove within or external to the attachment tube 34" to form at least a region having an internal diameter 122. The internal diameter 122 provides a selected thickness relative to an external diameter of the attachment tube 34". The vibration reduction feature 120 may include a complete annular groove or may include formed struts or connections therein.

Moreover, the vibration reduction feature 120 may provide a selected flexibility at a selected position along a length of the attachment tube 34" to assist in mitigating or eliminating a selected vibration during the operation of the tool tip 20. For example, the vibration reduction feature may have a length 120a. The vibration reduction feature 120 may have a first end 120' spaced a distance 120b from the proximal end 35 and a second end 120" spaced a distance 120c from the distal end 120c. The selected lengths of 120a, 120b, and 120c can assist in tuning the dampening feature and may be selected based upon characteristics of the tube 34" and or the tool 20 and or the attachment base 30. Further, the relationship of the internal diameters 122 and 94 may be further selected to tune the dampening amount. The internal diameters 122 and 94 and sections having them may also be selectively placed along the length of the attachment tubes 34, 34', and 34".

As discussed above, the bearing 100 may be positioned within the attachment tube 34" and operation of the tool 20 rotating around the axis 20a may cause vibration in the attachment assembly 40. The vibration reduction feature 120 including the internal diameter 122 relative to the internal diameter 94 of the attachment tube 34" can provide a selected reduction of the vibration. The attachment tube 34" can be connected with the attachment base 30 in a manner substantially similar to that illustrated in FIG. 3A, 3B, or 3C, either directly to the attachment base 30, interconnection through the damping member 70 only, or a combination of interconnection to both a damping member 70 and direct attachments to the attachment base 30.

Accordingly, it is understood that the attachment tube 34" can be formed to include a vibration reduction or tuning feature such as the feature 120, illustrated in FIG. 5, or a thickness 90 of a wall as illustrated in FIGS. 3A, 3B, or 3C, either alone or in combination with a damping member 70. The vibration reduction features can assist in tuning vibration, which may include minimizing or eliminating vibration, due to operation of the instrument assembly 10 to rotate the tool 20 around the axis 20a. By reducing the vibration, unselected movements of the instrument may be reduced and operation of the tool 20 may be smoother. This can allow for substantially precise operation of the tool 20, especially over long periods of time, to allow for speed of an operation and selected outcomes for a patient. Reducing vibration may also reduce user fatigue.

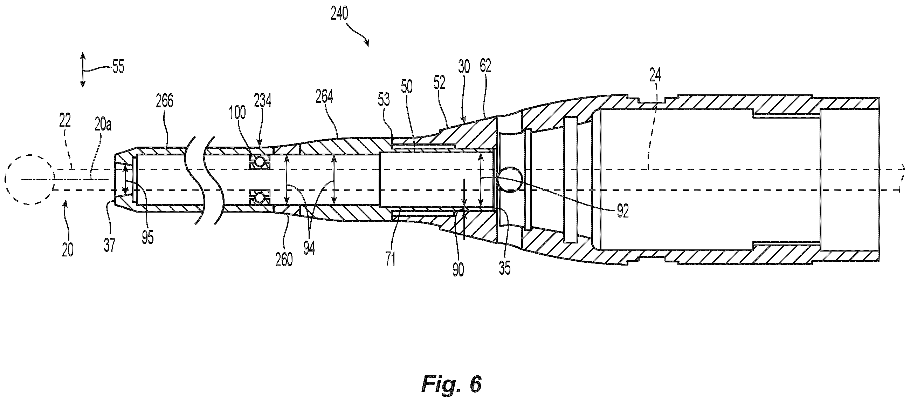

With reference to FIG. 6 an attachment assembly 240 is illustrated. The attachment assembly 240 is similar to the attachment assembly 40, as discussed above. The attachment assembly 240, illustrated in cross-section FIG. 6, is understood to include the bore which may include the internal diameter 92 and the second and different internal diameter 94. Further, the attachment assembly 240 may include the attachment base 30, as discussed above, and an attachment tube 234 connected to the attachment base 30 in at least one of various manners. The attachment tube 234 may be (1) only connected directly to the attachment base 30, (2) interconnected with a damping member (not illustrated in FIG. 6) which is connected to the attachment base 30, or (3) connected to both a damping member and the attachment base 30 (not specifically illustrated in FIG. 6) according to various embodiments including features of those embodiments discussed above. The attachment assembly 240 can further include the connection portion 52 of the attachment base 30 and the connection portion 50 of the attachment tube 234.

The attachment tube 234 may include various interconnected portions, such as a damping member 260 that interconnects a first rigid member 264 and a second rigid member 266. The rigid members 264, 266 can include selected exterior dimensions, including those discussed above, and further include the internal diameters 92 and/or 94. Further, the damping member 260 can also define the internal diameter 94. It is understood, however, that the damping member 260 can have a selected axial position on the attachment tube 234, mass, length, density, internal diameter, or the like, as discussed above. Further, the damping member 260 can be formed of various materials, including those discussed above.

The attachment tube 234 may be connected to the attachment base 30, as discussed above. However, the damping member 260 may be included as a feature of the attachment tube 234 or between a portion of the attachment tube 234 and the attachment base 30. As illustrated in FIG. 6, the attachment base 30 can connect with the first rigid portion 264 and the damping member 260 can be positioned between a portion of the attachment tube 234, including the second rigid portion 266, and the attachment base 30. Therefore the damping member 260 is positioned between at least a portion of the attachment tube 234 (i.e., the second rigid member 266) and the attachment base 30. In various configurations, one skilled in the art may consider the first rigid portion 264, when connected with the attachment base 30, a portion of the attachment base 30. However, it is understood, that another damping member, such as the damping member 70, may be positioned generally near the connection and areas 50, 52 such as in the gap 71. Therefore, it is further understood, that the attachment assembly 240, according to various embodiments, may include a plurality of damping members. As specifically illustrated in FIG. 6, the damping member 260 is positioned a distance from the distal end 53 of the attachment base 30 and is completely integrated into the attachment tube 234.

The damping member 260 may be connected to the rigid members 264, 266 according to various appropriate bonding techniques. For example, the damping member 260 may be adhered, welded, directly molded onto the rigid members 264, 266, or other appropriate bonding or fixation techniques. Nevertheless, the damping member 260 may dampen motion between the second rigid member 266 and the first rigid member 264 and the attachment base 30. Therefore, the damping member 260, including various features of the damping member 260, may be used to assist in tuning chatter and vibration of an instrument 10, as discussed above.

The vibration reduction features can be tuned individually or collectively to specific configurations of the tool assembly 10, as noted above. For example, configurations of the tool assembly may include the size of the bore, length of the attachment tube 34, angle of the attachment tube 34', etc. The tuning feature(s) may include selecting a thickness 90 of the wall, a length of the region having the thickness 90, an axial position of the wall with the thickness 90, thickness and/or length or axial positioning of the damping member 70, and/or axial position, length, or relative internal diameter of the dampening feature 120.

Vibration responses and associated reductions (e.g., by damping or increases in stiffening or decreases in stiffening) of the tool 20 and attachment assembly 40 can be modeled by one or more Structural Dynamic techniques. The modeling techniques may include modal analysis, harmonic analysis, or transient dynamic analysis. Also, or alternatively, various physical testing techniques may be used to determine the vibration responses. These methods may predict the frequencies and displacements involved as appropriate to the technique.

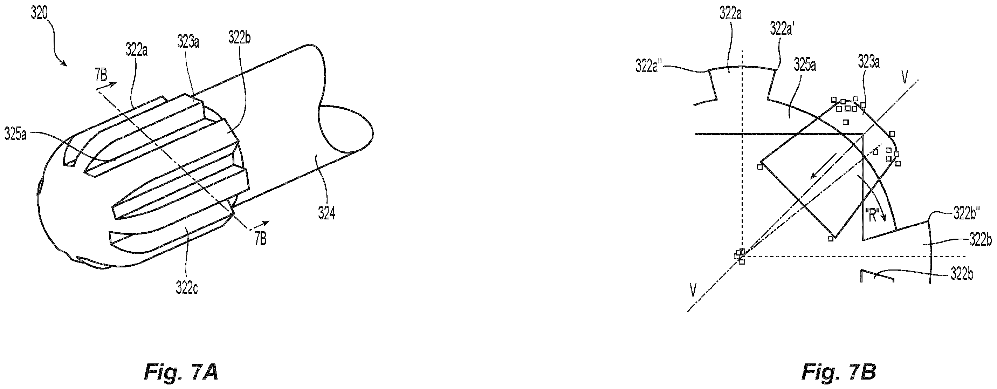

If tuned correctly, a controlled amount of tool chatter may be effective in improving cutting efficiency or improving cutting effectiveness. For example and as illustrated in FIGS. 7A and 7B, if the amount of chatter of the surgical bur 320 contacting the bone is effectively controlled and properly tuned, the excess potential energy associated with each cutting flute 320 as it contacts the bone will improve the effectiveness of the cutting action through the cutting stroke.

More particularly and as best seen in the greatly-enlarged view of FIG. 7B, a push-off element, e.g., 323a, is disposed in a clearance surface 325, e.g., clearance surface 325a between each pair of adjacent cutting flutes, e.g., cutting flutes 322a and 322b. As the surgical bur 320 rotates, each push-off element, e.g., push off element 323a disposed between the respective flutes 322a and 322b, deflects or "bumps" the surgical bur 320 away from the bone by a small or controlled amount (e.g., dependent up the proportions and dimensions of the push-off element 323a, rotational speed of the surgical bur 320, depth of the clearance surface 325, stiffness of the stem 324, inter alia) creating potential energy which is utilized and timed to increase cutting efficiency and effectiveness. In other words, prior the next flute, e.g., flute 322b, contacting the bone for cutting purposes during the rotational cut, the stiffness of the system and tuned potential energy provided by the push-off element 323a (as described above) snaps the surgical bur 320 back into and towards the bone forcing the flute 322b to make a more aggressive and efficient cut.

As can be appreciated, instead of relying on the surgeon to pre-tune or continually-tune the instrument assembly 10 during use to minimize tool chatter or rely on a surgeon's skill to try and more finely tune the instrument assembly 10 "on the fly" to more effectively and more efficiently cut bone using tool chatter, customizing the surgical bur 320 with push-off elements, e.g., push-off elements 323a-323b, allows the surgical bur 320 to "self-tune" as long as the instrument assembly 10 remains within pre-designed operating parameters, e.g., operating speed (RPM), torque, etc.

In embodiments, the dimensions of the push-off element (hereinafter push-off element 323a) may be designed depending upon a particular surgical purpose or to match the particular cutting characteristics of the cutting flute 322. In embodiments, the push-off element 323a is non-cutting but may include other features to enhance a cutting effect, e.g., scoring, rasping, etc. In embodiments, the geometry of the push-off element 323a may be synchronized such that the surgical bur 320 does not completely bottom out as the approaching cutting flute 322 comes into contact with the bone resulting in a more angular or shaved cut versus an aggressive cut. In this instance, the push-off element 323a might be dimensioned to push-off from the bone more along the direction of rotation "R" than along the vertical axis "V-V".

In other embodiments, more than one push-off element 323a may be disposed between adjacent flutes, e.g., flutes 322a and 322b. The spacing of the push-off elements 323a, 323b disposed within the clearance surfaces 325a, 325b between the adjacent flute pairs, e.g., flutes 322a, 322b and flutes 322b, 322c, respectively, may be the same or different depending upon a particular purpose or to achieve a particular tuning effect.

As mentioned above, the tuning of the tool chatter is dependent upon many factors which may be designed to allow the surgical bur 320 to "self-tune" at a specific speed (RPM) and/or torque thereby reducing the manual tuning by the surgeon during use. These factors may be utilized to enhance the effectiveness and efficiency of the cutting effect and efficiency in conjunction with the push-off element 323a. Such factors include: the quantity and mass of the cutting flutes 322, the geometry and mass of the tool stem 324, e.g., cross-sectional properties, the unsupported length of the tool stem 324 distal to the bearings 100, the quantity and location of the tool stem support bearings 100 along the attachment length, the geometry and lateral stiffness (mass) of the attachment tube, e.g., attachment tube 34, etc.

Moreover and as mentioned above, designs to the geometry and mass of the surgical bur 320, geometry and mass of the flutes 322, geometry and mass of the tool stem 324 and/or geometry and mass of the attachment tube 34, may also be utilized to control the other vibrational characteristics of the instrument assembly 10 including torsional vibrational effects on other system components during activation. In embodiments, the tool stem 324 or the flutes 322 may be provided with additional mass to improve the stability, stiffness or smoothness/resonance of the surgical bur 320.

It has been shown that utilizing a series of push-off elements, e.g., push-off elements 323a-323b, between adjacent flutes 322a, 322b with a surgical bur 320 running within proper manufacturing parameters improves cutting efficiency (measured by the amount of material cut normalized by reaction force to bone fed into the surgical bur) as much as 2 to 3 times over other known prior art designs.

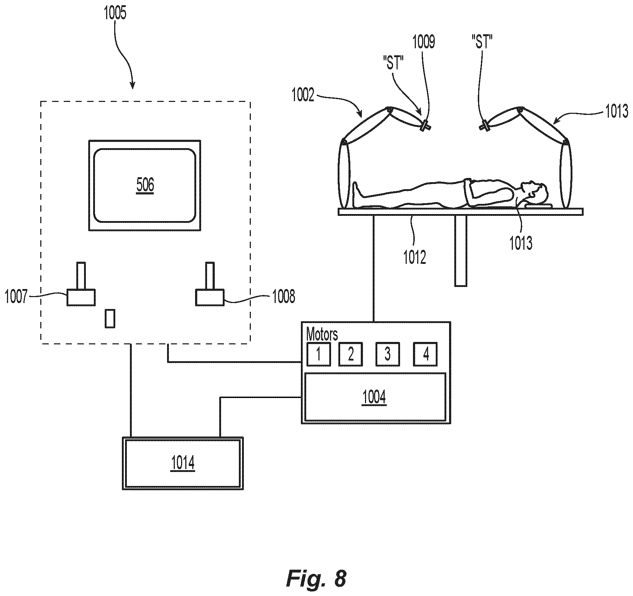

Turning to FIG. 8, a robotic surgical system 1000 configured for use in accordance with the present disclosure is shown. Aspects and features of robotic surgical system 1000 not germane to the understanding of the present disclosure are omitted to avoid obscuring the aspects and features of the present disclosure in unnecessary detail.

Robotic surgical system 1000 generally includes a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a clinician, e.g., a clinician, may be able to telemanipulate robot arms 1002, 1003 in a first operating mode. Robotic surgical system 1000 may be configured for use on a patient 1013 lying on a patient table 1012 to be treated in a minimally invasive manner. Robotic surgical system 1000 may further include a database 1014, in particular coupled to control device 1004, in which are stored, for example, pre-operative data from patient 1013 and/or anatomical atlases.

Each of the robot arms 1002, 1003 may include a plurality of members, which are connected through joints, and a mounted device which may be, for example, a surgical tool "ST." The surgical tools "ST" may include, for example, the micro-burring instrument assemblies 10 of the present disclosure, thus providing any of the above-detailed functionality on a robotic surgical system 1000.

Robot arms 1002, 1003 may be driven by electric drives, e.g., motors, connected to control device 1004. The motors, for example, may be rotational drive motors configured to provide rotational inputs to accomplish a desired task or tasks. Control device 1004, e.g., a computer, may be configured to activate the motors, in particular by means of a computer program, in such a way that robot arms 1002, 1003, and, thus, their mounted surgical tools "ST" execute a desired movement and/or function according to a corresponding input from manual input devices 1007, 1008, respectively. Control device 1004 may also be configured in such a way that it regulates the movement of robot arms 1002, 1003 and/or of the motors.

Control device 1004, more specifically, may control one or more of the motors based on rotation, e.g., controlling to rotational position using a rotational position encoder (or Hall effect sensors or other suitable rotational position detectors) associated with the motor to determine a degree of rotation output from the motor and, thus, the degree of rotational input provided. Alternatively or additionally, control device 1004 may control one or more of the motors based on torque, current, or in any other suitable manner.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects. Those skilled in the art will envision other modifications within the scope and spirit of the claims appended hereto.

## Claims

1. A surgical bur for use in cutting bone, comprising:
a stem adapted to selectively couple to an attachment tube of a micro-burring instrument assembly and configured for selective rotation upon activation of the micro-burring instrument;
a plurality of cutting flutes disposed at a distal end of the stem, the plurality of cutting flutes defining a corresponding number of clearance surfaces disposed therebetween, each cutting flute of the plurality of cutting flutes including a cutting edge and a trailing edge; and
at least one push-off element defined in the clearance surface between at least one adjacent pair of cutting flutes of the plurality of cutting flutes, the at least one push-off element configured to contact bone during rotation of the surgical bur and configured to deflect the surgical bur relative thereto and optimize the cutting efficiency and effectiveness of the surgical bur based on at least the rotational speed of the surgical bur.

2. The surgical bur for use in cutting bone according to claim 1, wherein a corresponding plurality of push-off elements are disposed between the plurality of cutting flutes.

3. The surgical bur for use in cutting bone according to claim 2, wherein the plurality of push-off elements is configured to deflect the surgical bur based on at least an angle of rotation of the surgical bur or an angle of oscillation of the surgical bur.

4. The surgical bur for use in cutting bone according to claim 2 or 3, wherein the plurality of push-off elements is non-cutting.

5. The surgical bur for use in cutting bone according to one of claims 2-4, wherein a series of push-off elements are disposed between at least one adjacent pair of cutting flutes of the plurality of cutting flutes.

6. The surgical bur for use in cutting bone according to claim 5, wherein the series of push-off elements disposed between at least one adjacent pair of cutting flutes of the plurality of cutting flutes are equally or unequally spaced between cutting flutes.

7. The surgical bur for use in cutting bone according to one of claims 2-6, wherein the plurality of push-off elements disposed between the adjacent pairs of cutting flutes of the plurality of cutting flutes are synchronized such that the surgical bur does not completely bottom out as the next approaching cutting flute in rotation comes into contact with bone.

8. The surgical bur for use in cutting bone according to one of claims 2-7, wherein the plurality of push-off elements disposed between the adjacent pairs of cutting flutes of the plurality of cutting flutes are synchronized such that the surgical bur pushes off from the bone between each adjacent pair of flutes more laterally along the direction of rotation than vertically along a direction normal to the bone.

9. A micro-burring instrument for cutting bone in combination with a surgical bur, optionally a surgical bur according to one of claims 1-8, the micro-burring instrument comprising:
a micro-burring assembly including:
a motor housing having a motor disposed therein;
an attachment tube operably coupled at a proximal end thereof to the motor housing and configured to selectively receive the surgical bur therein for use in cutting bone; and
a collet for operably receiving a stem of the surgical bur such that the surgical bur is selectively rotatable upon activation of the motor, wherein the surgical bur includes:
a plurality of cutting flutes disposed at a distal end of the stem, the plurality of cutting flutes defining a corresponding number of clearance surfaces disposed therebetween, each cutting flute of the plurality of cutting flutes including a cutting edge and a trailing edge; and
at least one push-off element defined in the clearance surface between at least one adjacent pair of cutting flutes of the plurality of cutting flutes, the at least one push-off element configured to contact bone during rotation of the surgical bur and configured to deflect the surgical bur relative thereto and optimize the cutting efficiency and effectiveness of the surgical bur based on at least the rotational speed of the surgical bur.

10. The micro-burring instrument for use in cutting bone according to claim 9, wherein a corresponding plurality of push-off elements are disposed between the plurality of cutting flutes.

11. The micro-burring instrument for use in cutting bone according to claim 10, wherein the plurality of push-off elements is configured to deflect the surgical bur based on at least an angle of rotation of the surgical bur or an angle of oscillation of the surgical bur.

12. The micro-burring instrument for use in cutting bone according to claim 10 or 11, wherein the plurality of push-off elements is non-cutting.

13. The micro-burring instrument for use in cutting bone according to one of claims 10-12, wherein a series of push-off elements are disposed between at least one adjacent pair of cutting flutes of the plurality of cutting flutes, wherein, optionally, the series of push-off elements disposed between at least one adjacent pair of cutting flutes of the plurality of cutting flutes are equally or unequally spaced between cutting flutes.

14. The micro-burring instrument for use in cutting bone according to one of claim 10-13, wherein the plurality of push-off elements disposed between the adjacent pairs of cutting flutes of the plurality of cutting flutes are synchronized such that the surgical bur does not completely bottom out as the next approaching cutting flute in rotation comes into contact with bone.

15. The micro-burring instrument for use in cutting bone according to one of claims 10-14, wherein the plurality of push-off elements disposed between the adjacent pairs of cutting flutes of the plurality of cutting flutes are synchronized such that the surgical bur pushes off from the bone between each adjacent pair of flutes more laterally along the direction of rotation than vertically along a direction normal to the bone.
